(19) Europäisches Patentamt
European Patent Office
Office européen des brevets

(11) Veröffentlichungsnummer: **0 301 398**
**A2**

# (12) EUROPÄISCHE PATENTANMELDUNG

(21) Anmeldenummer: **88111706.3**

(51) Int. Cl.4: **C07K 5/06 , C07K 1/02**

(22) Anmeldetag: **20.07.88**

(30) Priorität: **30.07.87 CH 2919/87**

(43) Veröffentlichungstag der Anmeldung:
**01.02.89 Patentblatt 89/05**

(84) Benannte Vertragsstaaten:
**AT BE CH DE ES FR GB IT LI LU NL SE**

(71) Anmelder: **LONZA AG**

**Gampel/Wallis(CH)**

(72) Erfinder: **Meul, Thomas, Dr.**
**Balfrinstrasse 21**
**VISP (Kanton Wallis)(CH)**
Erfinder: **Duc, Laurent, Dr.**
**Rue de la Meunière**
**SION (Kanton Wallis)(CH)**

(74) Vertreter: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**D-8000 München 40(DE)**

## (54) Verfahren zur Herstellung von N-[(4-Hydroxy-pyrrolidin-2-on-l-yl)-acetyl]-glycinamid.

(57) Es wird ein neues 4-Hydroxy-2- pyrrolidinonderivat und ein Verfahren zu dessen Herstellung beschrieben

EP 0 301 398 A2

## Verfahren zur Herstellung von N-[(4-Hydroxy-pyrrolidin-2-on-1-yl)-acetyl]-glycinamid

Die Erfindung betrifft ein neues Verfahren zur Herstellung von N-[(4-Hydroxy-pyrrolidin-2-on-1-yl)-acetyl]-glycinamid, ein Analogon zum cerebral wirksamen Oxiracetam (4-Hydroxy-pyrrolidin-2-on-1-yl-acetamid), letzteres bekannt aus Pifferi et al, Il Farmaco, Ed.Sc. 1977, 32, 602.

Aus EP-A 0 207 681 sind zwei Verfahren bekannt, N-[(4-Hydroxy-pyrrolidin-2-on-1-yl)-acetyl]-glycinamid herzustellen.

Gemäss dem ersten Verfahren wird ein 2-Methylpropyl-3-carbamoylmethyl-beta-hydroxy-2-(1-methylethyl)-4-oxo-1-imidazolidinbutansäureester in Gegenwart von Wasser intramolekular cyclisiert zum Endprodukt.

Gemäss dem zweiten Verfahren wird dazu 4-Hydroxy-2-oxo-1-pyrrolidinessigsäure mit Glycinamid umgesetzt.

Beide Verfahren zeichnen sich in nachteiliger Weise durch sehr umständliche Verfahrensweisen aus, die im grosstechnischen Massstab nicht denkbar sind.

Es bestand daher die Aufgabe, ein neues grosstechnisch einsetzbares und einfaches Verfahren zu finden, das nicht mit den genannten Nachteilen behaftet ist.

Die Aufgabe konnte gelöst werden mit einem Verfahren durch Umsetzung

a) eines 4-Halogen-3-alkoxy-but-2E-ensäurealkylesters mit einem Glycinglycinalkylester zu einem N-[(4-Alkoxy-3-pyrrolin-2-on-1-yl)-acetyl]-glycinalkylester,

b) Weiterumsetzung mit Benzylalkohol, in Gegenwart einer Säure, intermediär zum N-[(4-Benzyloxy-3-pyrrolin-2-on-1-yl)-acetyl]-glycinalkylester,

c) Amidierung mit Ammoniak zum N-[(4-Benzyloxy-3-pyrrolin-2-on-1-yl)-acetyl]-glycinamid,

d) Abspaltung der Benzylschutzgruppe durch Hydrogenolyse in Gegenwart eines Palladiumkatalysators mit Wasserstoff und schliesslich

e) Reduktion mit einem komplexen Hydrid.

### Stufe a)

Der als Ausgangsprodukt verwendete 4-Halogen-3-alkoxy-but-2E-ensäurealkylester kann zweckmässig nach EP-A 0 216 324 auf einfache Weise hergestellt werden.

Vorteilhaft werden als Ausgangsprodukt die 4-Chlor-3-($C_1$-$C_2$)-alkoxy-but-2E-ensäure-($C_1$-$C_4$)-alkylester verwendet, besonders bevorzugt aber der 4-Chlor-3-methoxy-but-2E-ensäuremethylester.

Als Glycinglycinalkylester wird vorzugsweise ein ($C_1$-$C_4$)-niederalkylester, besonders bevorzugt der Ethylester, üblicherweise in Form des Hydrochlorids, eingesetzt.

Die Umsetzung verläuft zweckmässig in Gegenwart eines tertiären Amins, vorzugsweise eines Trialkylamins, wie Triethylamin, bei Temperaturen zwischen 50 und 80° C, vorteilhaft bei 80° C. Es ist ebenso von Vorteil, in cyanidierten Kohlenwasserstoffen, bevorzugt in Acetonitril, als Lösungsmittel zu arbeiten. Der nach einer Reaktionszeit von 4 bis 8 Stunden anfallende N-[(4-Alkoxy-3-pyrrolin-2-on-1-yl)-acetyl]-glycinalkylester kann auf laborübliche Weise aus dem Reaktionsgemisch isoliert und gegebenenfalls gereinigt werden.

### Stufen b und c

Zweckmässig werden die Stufen b und c ohne Isolierung des Zwischenproduktes nach Stufe b in einem Schritt durchgeführt. Selbstverständlich kann aber auch stufenweise mit Isolation des Zwischenproduktes nach b) verfahren werden.

Der Benzylalkohol wird vorteilhaft in einem Ueberschuss von 3 bis 10 Mol pro Mol zum Edukt aus Stufe a) zugesetzt. Dabei fungiert der Benzylalkohol gleichzeitig als Lösungsmittel für die Stufe b).

Als Säure wird in katalytischen Mengen von zweckmässig 5 bis 20 Mol% eine Sulfonsäure, wie z.B. Methansulfonsäure oder p-Toluolsulfonsäure zugesetzt.

Die Umsetzungstemperatur für die Einführung der Benzylschutzgruppen liegt zweckmässig zwischen 80 und 120° C, vorzugsweise zwischen 90 und 100° C.

Mit Vorteil wird die Reaktion bei einem leichten Unterdruck von 5 bis 100 mbar gefahren. Dadurch wird gewährleistet, dass die niedrig siedenden abgespaltenen Alkohole bei dieser Umsetzung aus dem Gleichgewicht entfernt werden.

Nach einer Reaktionszeit von ca. 4 Stunden kann der intermediär vorliegende Benzyloxypyrrolinester direkt, ohne Isolierung amidiert werden. Zweckmässig wird dazu die Reaktionslösung, gegebenenfalls nach zusätzlicher Verdünnung, vorzugsweise mit einem Alkohol, wie z.B. Methanol, mit Ammoniak gesättigt.

Bei Temperaturen von 20 bis 80° C kann die Reaktion nach ca. 8 bis 16 Stunden als beendet betrachtet werden.

Nach laborüblicher Aufarbeitung und gegebenenfalls nach zusätzlicher Reinigung kann das N-[(4-Benzyloxy-3-pyrrolin-2-on-1-yl)-acetyl]-glycinamid erhalten werden.

Stufen d und e

Zweckmässig werden die Stufen d und e in einem Schritt durchgeführt, ohne das Zwischenprodukt nach Stufe d) zu isolieren. Die Abspaltung der Benzylschutzgruppen geschieht in Gegenwart eines Palladiumkatalysators in Mengen von 5 bis 20 Gew.%, bezogen auf eingesetztes Edukt, aufgebracht auf einem üblichen Träger, vorteilhaft auf Kohle, mit Wasserstoff.

Zweckmässig arbeitet man bei Temperaturen zwischen 0 und 30° C und Drücken zwischen 1 und 10 bar.

Es ist von Vorteil, in einem aprotischen Lösungsmittel mit hoher Dielektrizitätskonstante, wie Dimethylacetamid oder Dimethylformamid zu arbeiten.

Nach 1 bis 5 Stunden kann die Hydrogenolyse als beendet betrachtet werden.

Das Zwischenprodukt N-[(2,4-Dioxo-pyrrolidin-1-yl)-acetyl]-glycinamid wird vorzugsweise nicht isoliert, zweckmässig wird unmittelbar mit einem komplexen Hydrid zum Endprodukt weiter reduziert. Als komplexe Hydride finden zweckmässig die Alkaliborhydride, vorteilhaft aber das Natriumborhydrid, Anwendung.

Die Umsetzungstemperatur wählt man zweckmässig zwischen 0 und 30° C.

Aus dem Reaktionsgemisch kann nach üblichem Aufarbeiten das Endprodukt N-[(4-Hydroxy-pyrrolidin-2-on-1-yl)-acetyl]-glycinamid, gegebenenfalls nach Reinigung, als weisses kristallines Produkt erhalten werden.

**Beispiel**

a) Herstellung von N-[(4-Methoxy-3-pyrrolin-2-on-1-yl)-acetyl]-glycinethylester

25,6 g (0,13 Mol) Glycinglycinethylester-hydrochlorid wurden in 60 ml Acetonitril suspendiert. Dazu gab man bei Rückfluss 13,2 g (0,13 Mol) Triethylamin. Anschliessend wurden nacheinander, in mehreren Portionen innerhalb von 4 Stunden 17,2 g (0,1 Mol) 4-Chlor-3-methoxy-but-2E-ensäuremethylester und 10,2 g (0,1 Mol) Triethylamin zugetropft.

Man destillierte das Lösungsmittel am Rotationsverdampfer ab und gab 150 ml Wasser zum Rückstand. Die wässrige Lösung wurde 4 mal mit je 50 ml Methylenchlorid extrahiert.

Die Methylenchlorid-Phase wurde separiert, über Natriumsulfat getrocknet und eingedampft.

Man erhielt 21,9 g Rohprodukt.

Eine Probe, die aus Toluol umkristallisiert wurde, hatte einen Smp. von 87,5 bis 88,5° C.

b)c) Herstellung von N-[(4-Benzyloxy-3-pyrrolin-2-on-1-yl)-acetyl]-glycinamid

4,7 g (18,4 mMol) N-[(4-Methoxy-3-pyrrolin-2-on-1-yl)-acetyl]-glycinethylester wurden in 10,8 g (100 mMol) Benzylalkohol gelöst und mit 0,1 g (1 mMol) Methansulfonsäure versetzt. Diese Reaktionsmischung wurde 4 Stunden bei 100° C und bei 20 mbar gerührt. Anschliessend gab man 20 ml Methanol zu und sättigte die Reaktionslösung mit gasförmigem Ammoniak. Man liess bei 60° C über Nacht rühren und destillierte anschliessend Methanol und Benzylalkohol im Vakuum ab. Der Rückstand wurde aus Methanol umkristallisiert.

Man erhielt 4,5 g N-[(4-Benzyloxy-3-pyrrolin-2-on-1-yl)-acetyl]-glycinamid. Smp. 169 bis 170° C.

$C_{15}H_{17}N_3O_4$ (303,32)

| | | |
|---|---|---|
| ber. C 59,4 | H 5,6 | N 13,9 |
| gef. C 59,0 | H 5,7 | N 14,0 |

d)e) Herstellung von N-[(4-Hydroxy-pyrrolidin-2-on-1-yl)-acetyl]-glycinamid

4,0 g (13,2 mMol) N[(4-Benzyloxy-3-pyrrolin-2-on1-yl)-acetyl]-glycinamid wurden in 50 ml Dimethylacetamid gelöst und mit 0,4 g Palladium auf Kohle 5% versetzt.

Diese Mischung wurde 4 Stunden bei 4 bar und Raumtemperatur mit Wasserstoff hydrogenolysiert. Anschliessend wurde der Katalysator abgenutscht und die Reaktionslösung bei 15° C innerhalb von 1 Stunde zu einer Lösung von 0,36 g (9,5 mMol) Natriumborhydrid in 25 ml Dimethylacetamid getropft.

Man liess 1 Stunde bei Raumtemperatur nachrühren und säuerte die Reaktionslösung mit einer Mischung aus 0,65 g Ameisensäure und 25 ml Methanol an. Die Lösungsmittel wurden im Wasserstrahlvakuum abgedampft und der Rückstand wurde in 30 ml Wasser aufgenommen.

Die wässrige Lösung wurde über 40 g stark sauren Kationenaustauscher und danach über 40 g schwach basischen Anionenaustauscher gegeben. Anschliessend wurde die wässrige Lösung mit 0,5 g Aktivkohle behandelt.

Nach Abnutschen der Aktivkohle und Eindampfen der wässrigen Lösung erhielt man 2,0 g weisses kristallines Produkt mit einem Smp. 153-154° C.

$C_8H_{13}N_3O_4$ (215,21)

ber. C 44,7 H 6,1 N 19,5
gef. C 44,6 H 6,3 N 19,1
$^1$H-NMR: (DMSO-$d_6$, 300 MHz) $\delta$ in ppm
8,10 (verbr.t, J = 5,8 Hz, 1H)
7,31 (verbr.s, 1H)
7,06 (verbr.s, 1H
5,21 (d, J = 4,0 Hz, 1H)
4,30 (m, 1H)
3,89 (d, J = 16,4 Hz, 1H)
3,77 (d, J = 16,4 Hz, 1H)
3,66 (d, J = 5,8 Hz, 2H)
3,62 (dd, J = 5,5 Hz, J = 10,6 Hz, 1H)
3,18 (dd, J = 2,5 Hz, J = 10,6 Hz, 1H)
2,57 (dd, J = 7,0 Hz, J = 16,6 Hz, 1H)
2,09 (dd, J = 2,8 Hz, J = 16,6 Hz, 1H)

**Ansprüche**

1. Verfahren zur Herstellung von N-[(4-Hydroxy-pyrrolidin-2-on-1-yl)-acetyl]-glycinamid der Formel

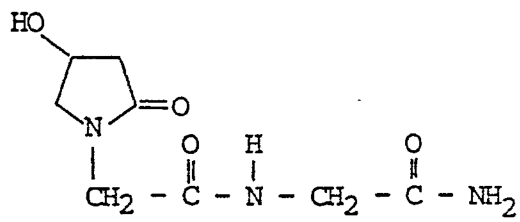

dadurch gekennzeichnet, dass man

a) einen 4-Halogen-3-alkoxy-but-2E-ensäurealkylester mit einem Glycinglycinalkylester zu einem N-[(4-Alkoxy-3-pyrrolin-2-on-1-yl)-acetyl]-glycinalkylester umsetzt,

b) weiter mit Benzylalkohol, in Gegenwart einer Säure, intermediär zum N-[(4-Benzyloxy-3-pyrrolin-2-on-1-yl)-acetyl]-glycinalkylester reagieren lässt, dann mit

c) Ammoniak zum N-[(4-Benzyloxy-3-pyrrolin-2-on-1-yl)-acetyl-glycinamid umsetzt und darauf

d) in Gegenwart eines Palladiumkatalysators mit Wasserstoff die Benzylschutzgruppen abspaltet und schliesslich

e) mit einem komplexen Hydrid zum Endprodukt reduziert.

2. Verfahren nach Patentanspruch 1, dadurch gekennzeichnet, dass man gemäss Schritt a) einen 4-Chlor-3-(C1-C2)-alkoxy-but-2E-ensäure-($C_1$-$C_4$)-alkylester bei Temperaturen von 50 bis 80 °C, in Gegenwart eines tertiären Amins, vorzugs weise Triethylamin, mit einem Glycinglycin-($C_1$-$C_4$)-alkylester, vorzugsweise als Hydrochlorid, zu einem N-[(4-($C_1$-$C_2$)-alkoxy-3-pyrrolin-2-on-1-yl)-acetyl]-glycin-($C_1$-$C_4$)-alkylester umsetzt.

3. Verfahren nach Patentansprüchen 1 und 2, dadurch gekennzeichnet, dass man die Umsetzung mit Benzylalkohol gemäss Schritt b), in Gegenwart einer Sulfonsäure, vorzugsweise Methansulfonsäure, bei Temperaturen zwischen 80 und 120 °C und Drücken zwischen 5 und 100 mbar durchführt.

4. Verfahren nach Patentansprüchen 1,2 und 3, dadurch gekennzeichnet, dass man den N-[(4-Benzyloxy-3-pyrrolin-2-on-1-yl)- acetyl]-glycinalkylester nicht isoliert, sondern direkt gemäss Schritt c) mit gasförmigem Ammoniak bei Temperaturen zwischen 20 und 80 °C zum entsprechenden Amid weiter reagieren lässt.

5. Verfahren nach Patentansprüchen 1,2,3 und 4, dadurch gekennzeichnet, dass man das N-[(4-Benzyloxy-3-pyrrolin-2-on-1-yl)-acetyl]-glycinamid gemäss Schritt d), in Gegenwart eines Palladiumkatalysators, mit Wasserstoff bei Temperaturen zwischen 0 und 30 °C hydrogenolysiert und schliesslich gemäss Schritt e) mit einem Alkaliborhydrid, vorzugsweise mit Natriumborhydrid, bei Temperaturen von 0 bis 30 °C zum Endprodukt hydriert.